# DEMANDE DE BREVET EUROPEEN

(11) **EP 3 572 038 A1**
(43) Date de publication de la demande: **27.11.2019**
(21) Numéro de dépôt: 19176338.2
(22) Date de dépôt: 24.05.2019
(51) Int. Cl.: A61D 19/02

(54) **DISPOSITIF DE COLLECTE DE SEMENCE ANIMALE**

(30) Priorité: 24.05.2018 FR 1854410
(71) Demandeur: IMV Technologies, 61300 Saint-Ouen-sur-Iton (FR)
(72) Inventeur: SCHMITT, Eric, 53700 VILLAINES-LA-JUHEL (FR); GORGES, Jean-Charles, 72610 CHENAY (FR)
(74) Mandataire: Santarelli

(57) **Abrégé**

Le dispositif (1) comporte un membre d'entrée (28) comportant une interface de connexion (40) à un vagin artificiel et délimitant une ouverture d'entrée (14), un filtre associé au membre d'entrée (28) et délimitant avec lui une chambre d'entrée (41) du côté d'une première face du filtre tournée vers l'ouverture (14), et un sachet de collecte (4) s'étendant entre une extrémité (8) fermée et une extrémité (7) présentant une ouverture (6) dans laquelle est engagé ledit membre d'entrée ; ledit sachet et ledit filtre délimitant une chambre de recueil (42) du côté de la seconde face (18) du filtre tournée vers l'extrémité (8) ; grâce à quoi de la semence entrant par l'ouverture (14) traverse le filtre et est recueillie dans la chambre de recueil (42) ; caractérisé en ce que le filtre est formé par une paroi ajourée (30) venue de matière avec le membre d'entrée.

## Description

### DOMAINE DE L'INVENTION

La présente invention a trait au domaine général de la collecte de semence animale, notamment la semence porcine, et plus précisément aux dispositifs pour collecter cette semence.

### ARRIERE-PLAN TECHNOLOGIQUE

On connaît de la demande internationale WO 2006/131781 un dispositif pour la collecte de semence animale, comprenant un cadre rigide sur lequel sont fixés une poche de filtration en textile non-tissé et un sachet de collecte, la poche de filtration étant positionnée à l'intérieur du sachet de collecte. Le cadre rigide comprend une partie d'adaptation pour assembler le dispositif avec un vagin artificiel et une armature qui maintient la forme de la poche de filtration sur toute sa hauteur. La partie d'adaptation a une géométrie adaptée à la sortie du vagin artificiel et délimite un passage pour l'écoulement de la semence animale depuis le vagin artificiel vers la poche de filtration. Le sachet de collecte présente une ligne de prédécoupe afin de pouvoir séparer la partie du sachet destinée à recueillir la semence de la partie du sachet fixée au cadre. La semence animale s'écoulant du vagin artificiel est filtrée par la poche puis recueillie dans le sachet. La filtration opérée par la poche permet de débarrasser la semence d'une substance indésirable formant une sorte de gel, appelée « tapioca ». Une fois recueillie dans le sachet de collecte, la semence animale est analysée dans une pièce spécifique souvent appelée laboratoire, puis elle est diluée, avant d'être conservée dans des sachets de conditionnement unitaires.

### OBJET DE L'INVENTION

L'invention vise à fournir un dispositif pour la collecte de semence animale qui soit plus performant que les dispositifs connus, tout en restant simple, commode et économique.

L'invention propose à cet effet un dispositif pour la collecte de semence animale, comportant :
- un membre d'entrée comportant une interface mécanique de connexion à un vagin artificiel et délimitant une ouverture d'entrée dans le dispositif de collecte ;
- un filtre associé au membre d'entrée et délimitant avec le membre d'entrée une chambre d'entrée s'étendant du côté d'une première face du filtre tournée vers l'ouverture d'entrée ; et
- un sachet de collecte s'étendant entre une zone d'extrémité distale fermée et une zone d'extrémité proximale présentant une ouverture dans laquelle est engagé ledit membre d'entrée ; avec ledit sachet qui est fixé audit membre d'entrée ; et avec ledit sachet et ledit filtre qui délimitent une chambre de recueil s'étendant du côté de la seconde face du filtre tournée vers la zone d'extrémité distale fermée du sachet ;
grâce à quoi de la semence entrant dans le dispositif par l'ouverture d'entrée traverse le filtre et est recueillie dans la chambre de recueil ;
caractérisé en ce que le filtre est formé par une paroi ajourée venue de matière avec le membre d'entrée.

Du fait que le filtre est formé par une paroi ajourée venue de matière avec le membre d'entrée, le dispositif selon l'invention est particulièrement simple et économique à fabriquer.

En particulier, pour réaliser le filtre, on économise la fourniture d'un textile non-tissé, dont l'intégration au reste du dispositif est par ailleurs une opération longue et coûteuse.

En outre, la pièce formant à la fois le filtre et le membre d'entrée peut être fabriquée de manière simple, par exemple moulée par injection.

Selon des caractéristiques simples, commodes et économiques du dispositif de collecte selon l'invention :
- ladite paroi ajourée présente une portion tubulaire s'étendant axialement entre une première extrémité tournée vers ladite zone d'extrémité proximale dudit sachet et une deuxième extrémité tournée vers ladite zone d'extrémité distale fermée dudit sachet, et une portion de fond s'étendant transversalement à ladite portion tubulaire et fermant cette dernière à sa deuxième extrémité ;
- ladite portion tubulaire a en section une forme circulaire ;
- ladite portion tubulaire présente une face externe nervurée ;
- ladite face externe nervurée présente des nervures droites orientées longitudinalement comme l'orientation axiale de la portion tubulaire ;
- lesdites nervures sont réparties autour de la portion tubulaire selon un pas angulaire prédéterminé ;
- ladite paroi ajourée est ajourée par une pluralité d'orifices ayant un diamètre minimal prédéterminé compris entre 0,5 mm et 1,5 mm, lesdits orifices étant répartis sur ladite paroi ajourée en étant agencés selon un motif prédéterminé procurant une densité surfacique d'orifices comprise entre 3 et 16 orifices par cm² ;
- ladite paroi ajourée est ajourée par une pluralité d'orifices et présente une portion tubulaire s'étendant axialement entre une première extrémité tournée vers ladite zone d'extrémité proximale dudit sachet et une deuxième extrémité tournée vers ladite zone d'extrémité distale fermée dudit sachet, et une portion de fond s'étendant transversalement à ladite portion tubulaire et fermant cette dernière à sa deuxième extrémité, lesdits orifices étant répartis sur ladite paroi ajourée en étant agencés sur ladite portion tubulaire selon un premier motif prédéterminé et sur ladite portion de fond selon un deuxième motif prédéterminé, lesdits premier motif prédéterminé et deuxième motif prédéterminé procurant une densité surfacique d'orifices plus importante sur ladite portion de fond que sur ladite portion tubulaire ;
- ledit premier motif prédéterminé procure une densité surfacique d'orifices sur la portion tubulaire comprise entre 3 et 7 orifices par cm² ;
- ledit deuxième motif prédéterminé procure une densité surfacique d'orifices sur la portion de fond comprise entre 10 et 16 orifices par cm² ;
- selon au moins l'un desdits premier motif prédéterminé et deuxième motif prédéterminé, lesdits orifices sont agencés en un quadrillage de rectangles ;
- selon au moins l'un desdits premier motif prédéterminé et deuxième motif prédéterminé, lesdits orifices sont agencés en quinconce ;
- chaque dit orifice est délimité par une surface en entonnoir augmentant de diamètre vers la seconde face du filtre ;
- ledit membre d'entrée et ladite paroi ajourée font partie d'une pièce en matière thermoplastique moulée par injection ; et/ou
- ledit dispositif comporte un sachet de protection qui recouvre ledit sachet de collecte et qui est fixé audit membre d'entrée.

### BREVE DESCRIPTION DES DESSINS

L'exposé de l'invention sera maintenant poursuivi par la description détaillée d'exemple de réalisation, donnée ci-après à titre illustratif et non limitatif, en référence aux dessins annexés. Sur ceux-ci :
- la figure 1 montre schématiquement un dispositif de collecte de semence animale selon l'invention, comportant un godet de filtration, un sachet de collecte dans lequel est partiellement reçu le godet de filtration et un sachet de protection recouvrant le sachet de collecte ;
- la figure 2 est la vue en coupe repérée II-II sur la figure 1 ;
- la figure 3 est une vue en perspective du godet de filtration pris isolément, montrant notamment le fond du godet ; et
- la figure 4 est une vue en coupe longitudinale du godet de filtration.

### DESCRIPTION DETAILLEE D'EXEMPLES DE REALISATION

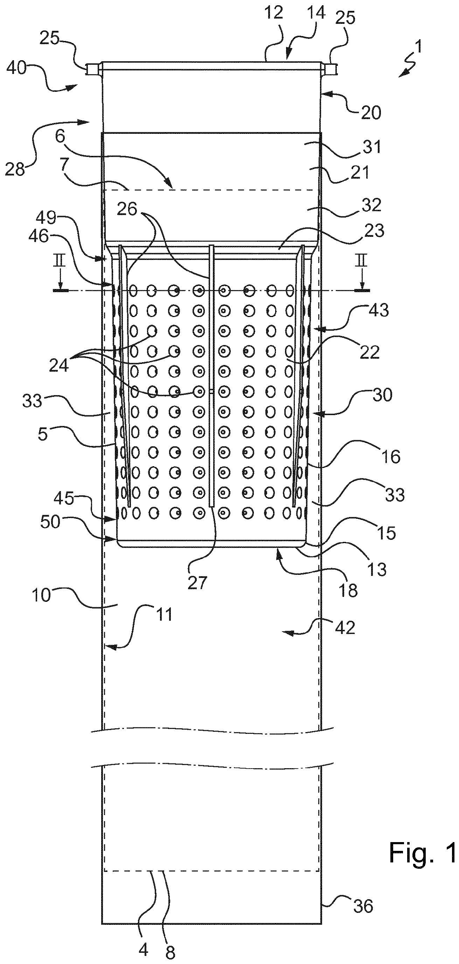

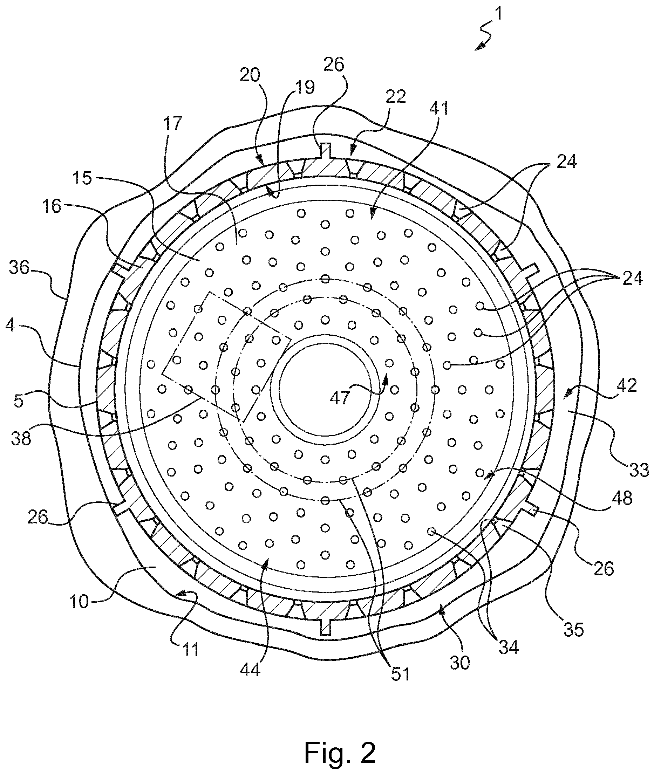

Le dispositif de collecte 1 de semence illustré sur les figures 1 à 4 comporte un sachet de collecte 4 (visible uniquement sur les figures 1 et 2) et un godet de filtration 5.

Le dispositif de collecte 1 est ici configuré pour être raccordé à un vagin artificiel (non illustré) afin de recueillir la semence s'en écoulant.

Le godet de filtration 5 est configuré pour débarrasser la semence entrant dans le dispositif de collecte 1 d'une substance indésirable formant une sorte de gel appelée « tapioca », que l'on retrouve notamment chez les porcins.

Le sachet de collecte 4 est quant à lui configuré pour recueillir et conditionner la semence une fois filtrée par le godet 5, c'est-à-dire la fraction liquide de la semence.

Le sachet de collecte 4 s'étend longitudinalement entre une zone d'extrémité proximale où il présente une extrémité proximale 7 tournée vers le haut sur la figure 1, et une zone d'extrémité distale où il présente une extrémité distale 8 tournée vers le bas sur la figure 1.

A son extrémité 7, le sachet 4 présente une ouverture 6, qui est donc ici tournée vers le haut, tandis que le fond du sachet 4 se situe à l'extrémité 8.

Le sachet de collecte 4 est ici formé par deux films en matière thermoplastique extrudés et soudés bord à bord, exceptés les bords situés à l'extrémité 7 de sorte à former l'ouverture 6.

Le fond du sachet 4 est ainsi formé par les bords soudés situés à l'extrémité 8.

Pour simplifier les dessins, les portions soudées des films ne sont pas représentées sur les figures 1 et 2.

Le sachet 4 présente au repos une forme généralement aplatie, les films ayant tendance à se plaquer l'une contre l'autre.

Lorsque le sachet 4 est mis en volume, il présente une forme généralement tubulaire, visible en section sur la figure 2, avec son espace interne 10 qui est délimité par sa face interne 11.

Le sachet 4 mis en volume présente en section transversale une forme qui est ici généralement circulaire.

Lorsque le sachet 4 est maintenu debout, comme montré sur la figure 1, la face interne 11 du sachet 4 s'étend généralement verticalement entre les extrémités 7 et 8.

Le godet de filtration 5 s'étend longitudinalement entre une extrémité 12 tournée vers le haut sur les figures 1, 3 et 4, et une extrémité 13 tournée vers le bas sur ces mêmes figures.

A son extrémité 12, le godet 5 présente une ouverture 14, qui est donc ici tournée vers le haut, tandis que le fond du godet 5 se situe à l'extrémité 13.

L'ouverture 14 forme une ouverture d'entrée pour la semence dans le dispositif de collecte 1.

Le godet 5 est ici réalisé en une matière thermoplastique comportant un polymère de type polyoléfine, ici plus précisément du polypropylène.

Le polypropylène est ici dans un état transparent, à 95%.

D'une manière générale, la matière formant le godet 5 est choisie de sorte à être suffisamment translucide ou transparente afin de pouvoir juger de la qualité de la semence (visualiser d'éventuelles impuretés ou traces de sang) et/ou visualiser le niveau de remplissage du godet 5 avec la semence.

Cette matière est en outre suffisamment rigide pour que le godet 5 puisse maintenir une forme prédéterminée stable, en présence d'efforts exercés sur lui.

Le godet 5 est ici formé d'une seule et même pièce moulée par injection. Par conséquent, toutes les parties que comprend le godet 5 sont venues de matière.

D'une manière générale, le godet 5 a une forme axisymétrique.

Le godet 5 comporte une paroi de fond 15 qui s'étend en regard de son ouverture 14 et une paroi latérale 16 qui s'étend depuis la paroi de fond 15, transversalement à cette dernière, jusqu'à l'ouverture 14 que cette paroi latérale 16 délimite.

La paroi de fond 15 forme ainsi le fond du godet 5.

L'épaisseur de la paroi de fond 15 et de la paroi latérale 16 est ici d'environ 2 mm.

En pratique, cette épaisseur peut être comprise entre 1 mm et 4 mm.

La paroi de fond 15 est ici plate et s'étend généralement horizontalement lorsque le godet 5 est maintenu debout comme montré sur les figures 1, 3 et 4.

On notera que la paroi de fond 15 présente une face interne 17 qui délimite l'espace interne au godet 5, et une face externe 18 opposée à la face interne 17.

La paroi latérale 16 a une forme généralement tubulaire et s'étend axialement selon une direction généralement verticale lorsque le godet 5 est maintenu debout comme montré sur les figures 1, 3 et 4.

La paroi latérale 16 présente en section transversale une forme ici généralement circulaire.

On notera que la paroi latérale 16 présente une face interne 19 qui délimite l'espace interne au godet 5, et une face externe 20 opposée à la face interne 19.

La paroi latérale 16 présente une portion supérieure 21, située du côté de l'ouverture 14 et délimitant cette dernière, une portion inférieure 22 située du côté de la paroi de fond 15 et raccordée à cette dernière, et une portion intermédiaire 23 raccordant la portion supérieure 21 à la portion inférieure 22.

Ainsi, la portion inférieure 22 s'étend axialement entre une première extrémité 49 tournée vers la zone d'extrémité proximale 7 du sachet 4 et raccordée à la portion intermédiaire 23, et une deuxième extrémité 50 tournée vers la zone d'extrémité distale 8 du sachet 4 et raccordée à la paroi de fond 15, laquelle paroi de fond 15 s'étend transversalement à la portion inférieure 22 en fermant cette dernière (c'est-à-dire en fermant l'espace interne qu'elle délimite).

On notera que d'une manière générale, le diamètre externe de la paroi latérale 16 diminue depuis l'ouverture 14 jusqu'à la paroi de fond 15.

La paroi latérale 16 présente ainsi une certaine conicité favorable au démoulage du godet 5 lors de sa fabrication.

On notera encore que cette diminution de diamètre est relativement faible dans la portion supérieure 21 et dans la portion inférieure 22 de sorte que ces portions 21 et 22 présentent un aspect quasiment cylindrique.

En revanche, cette diminution de diamètre est beaucoup plus prononcée dans la portion intermédiaire 23 que dans les portions supérieure 21 et inférieure 22, de sorte que la face externe 20 présente à cet endroit un épaulement.

D'une manière générale, on notera que le diamètre externe de la paroi latérale 16 est plus grand dans la portion supérieure 21 que dans la portion inférieure 22.

Ainsi, la partie de la face externe 20 située dans la portion inférieure 22 est globalement en retrait (vers l'intérieur du godet 5) par rapport à la partie de la face externe 20 située dans la portion supérieure 21.

Le diamètre externe moyen de la portion supérieure 21 est ici d'environ 54 mm.

Le diamètre externe moyen de la portion inférieure 22 est ici d'environ 49 mm.

La différence de diamètre externe moyen entre la portion supérieure 21 et la portion inférieure 22 est donc ici d'environ 5 mm.

A niveau de la portion intermédiaire 23 plus précisément, la différence de diamètre externe entre la portion supérieure 21 et la portion inférieure 22, c'est-à-dire la largeur de l'épaulement, est ici d'environ 3 mm.

Le godet 5 présente en outre une pluralité d'orifices 24, ici ménagés à la fois dans une région 43 de la portion inférieure 22 de la paroi latérale 16 et dans une région 44 de la paroi de fond 15.

Ces régions forment ainsi respectivement une région ajourée 43 de la paroi latérale 16 et une région ajourée 44 de la paroi de fond 15, en dehors desquelles la paroi latérale 16 et la paroi de fond 15 ne sont pas ajourées par les orifices 24.

La région 43 a une forme tubulaire et s'étend entre un bord inférieur 45 tourné vers la paroi de fond 15 et un bord supérieur 46 tourné vers la portion supérieure 21.

La région 44 a une forme d'anneau plat et s'étend radialement entre un bord interne 47 et un bord externe 48.

Les orifices 24 sont configurés afin de permettre à la semence de s'échapper du godet de filtration 5 tout en retenant dans ce dernier la substance « tapioca ».

En particulier, le diamètre minimal des orifices 24 est choisi afin de retenir la substance « tapioca ».

La portion inférieure 22 de la paroi latérale 16 et la paroi de fond 15 forment ainsi ensemble une paroi ajourée 30 configurée pour filtrer la semence, la portion inférieure 22 formant une portion tubulaire de la paroi ajourée 30 tandis que la paroi de fond 15 forme une portion de fond de la paroi ajourée 30.

Comme montré en particulier sur la figure 2, les orifices 24 sont chacun délimité par une surface présentant ici une portion cylindrique 34, de section ici circulaire, suivie d'une portion en entonnoir 35.

La portion cylindrique 34 est tournée vers l'intérieur du godet 5, tandis que la portion en entonnoir 35 est tournée vers l'extérieur du godet 5 et augmente de diamètre vers l'extérieur du godet 5 ; c'est-à-dire vers la face externe 20 pour les orifices 24 ménagés dans la paroi latérale 16, et vers la face externe 18 pour les orifices 24 ménagés dans la paroi de fond 15.

Le diamètre de la portion cylindrique 34 correspond au diamètre minimal de l'orifice 24, qui est ici d'environ 0,8 mm.

En pratique, ce diamètre minimal peut être compris entre 0,5 mm et 1,5 mm.

Le diamètre de la portion en entonnoir 35 augmente depuis le diamètre minimal jusqu'à atteindre un diamètre maximal qui est ici d'environ 2 mm.

D'une manière générale, dans chacune des régions 43 et 44 de la paroi ajourée 30, les orifices 24 sont répartis en étant agencés selon un motif prédéterminé.

Sur la paroi latérale 16, et plus précisément dans la région 43, les orifices 24 sont agencés selon un premier motif prédéterminé présentant une succession de colonnes, ici vingt-quatre colonnes, réparties autour de la portion inférieure 22 selon un pas angulaire prédéterminé d'environ 15 degrés.

Chaque colonne comporte ici douze orifices 24, le nombre total d'orifices 24 ménagés dans la portion inférieure 22 étant donc ici de 24 x 12 = 288 orifices.

Chaque colonne est orientée longitudinalement comme l'orientation axiale de la paroi latérale 16, c'est-à-dire ici sensiblement verticalement.

Entre deux colonnes voisines, les orifices 24 correspondants sont situés à la même hauteur (c'est-à-dire que ces orifices 24 sont alignés horizontalement), de sorte que, selon ce premier motif prédéterminé, les orifices 24 sont en outre globalement agencés en un quadrillage, ici plus précisément de rectangles (l'écart vertical entre deux orifices 24 adjacents d'une même colonne est ici plus faible que l'écart horizontal entre deux orifices 24 correspondants de deux colonnes voisines).

En variante, entre deux colonnes voisines, les orifices 24 correspondant pourraient être verticalement décalés de sorte que les orifices 24 seraient globalement agencés en quinconce.

Sur la paroi de fond 15, et plus précisément dans la région 44, qui a ici la forme d'un disque, les orifices 24 sont agencés selon un deuxième motif prédéterminé présentant une succession de cercles 51 concentriques, ici 8 cercles 51 (dont deux sont représentés en trait mixte sur la figure 2).

Chaque cercle 51 comporte ici seize orifices, à l'exception du cercle le plus externe qui comporte huit orifices de plus (soit vingt-quatre orifices en tout), le nombre total d'orifices 24 ménagés dans la paroi de fond 15 étant donc ici de 8 x 16 + 8 = 136 orifices.

Entre deux cercles 51 voisins, les orifices 24 correspondant présentent un décalage angulaire de sorte qu'ils ne sont pas alignés radialement et sont agencés en quinconce.

D'une manière générale, on notera qu'il est possible de caractériser les régions ajourées 43 et 44 de la paroi ajourée 30 par une densité surfacique d'orifices 24.

Pour une région ajourée donnée de la paroi ajourée 30, dans laquelle les orifices 24 sont agencés selon un motif prédéterminé donné, la densité surfacique d'orifices est ici définie par le nombre maximal d'orifices 24 que l'on peut entièrement inclure dans un centimètre carré (1 cm²) de surface de paroi ajourée 30, la surface considérée étant ici celle de la face interne de la paroi ajourée 30, c'est-à-dire celle délimitant l'espace interne du godet 5.

Cette face interne de la paroi ajourée 30 est ici formée par la face interne 17 de la paroi de fond 15 et par la partie de la face interne 19 de la paroi latérale 16 située sur sa portion inférieure 22.

En général, c'est-à-dire quelle que soit la région ajourée 43 ou 44 considérée, la densité surfacique d'orifices 24 sur la paroi ajourée 30 est comprise entre 3 et 16 orifices par cm².

On notera qu'ici, en particulier, le premier motif prédéterminé et le deuxième motif prédéterminé procurent une densité surfacique d'orifices 24 plus importante sur la paroi de fond 15 que sur la paroi latérale 16.

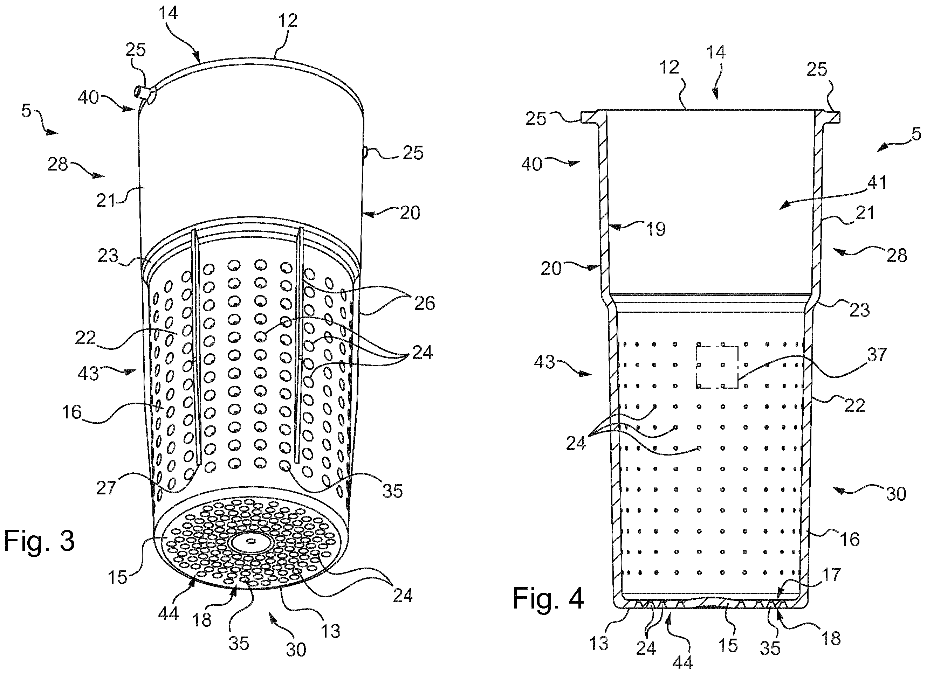

S'agissant de la région ajourée 43, une zone 37 représentant 1 cm² de surface interne de la paroi ajourée 30 est représentée sur la figure 4.

La zone 37 est positionnée de sorte à entièrement inclure un nombre maximal d'orifices, qui est ici de quatre orifices 24.

Le premier motif prédéterminé procure donc une densité surfacique d'orifices 24 sur la portion inférieure 22 de la paroi latérale 16 qui est ici de 4 orifices par cm².

D'une manière générale, la densité surfacique d'orifices 24 sur la portion inférieure 22 peut être comprise entre 3 et 7 orifices par cm².

S'agissant de la région ajourée 44, une zone 38 représentant 1 cm² de surface interne de la paroi ajourée 30 est représentée sur la figure 2.

La zone 38 est positionnée de sorte à entièrement inclure un nombre maximal d'orifices 24, qui est ici de douze orifices 24 (les orifices non entièrement inclus dans la zone 38 ne sont pas comptabilisés).

Le deuxième motif prédéterminé procure donc une densité surfacique d'orifices 24 sur la paroi de fond 15 qui est ici de 12 orifices par cm².

D'une manière générale, la densité surfacique d'orifices 24 sur la paroi de fond 15 peut être comprise entre 10 et 16 orifices par cm².

On notera qu'ici, le premier motif prédéterminé et le deuxième motif prédéterminé procurent une densité surfacique d'orifices uniforme respectivement sur l'ensemble de la région ajourée 43 de la paroi latérale 16 et sur l'ensemble de la région ajourée 44 de la paroi de fond 15.

En variante, le premier et/ou le deuxième motif prédéterminé pourrait procurer une densité surfacique d'orifices 24 qui serait variable selon le positionnement de la zone 37 et/ou 38 sur la région ajourée 43 et/ou 44 respective.

Dans ce cas, on définirait la densité surfacique d'orifices 24 comme étant une densité surfacique d'orifices 24 moyenne sur l'ensemble de la région 43 et/ou 44 considérée, calculée par exemple en divisant le nombre d'orifices 24 par la superficie de la surface interne de la paroi ajourée 30 dans la région 43 et/ou 44 considérée.

La face externe de la portion inférieure 22 est ici nervurée. Plus précisément, le godet 5 présente une pluralité de nervures 26 ménagées sur la portion inférieure 22, en saillie de la face externe 20.

Les nervures 26 sont ici au nombre de six et sont réparties autour de la portion inférieure 22 selon un pas angulaire prédéterminé d'environ 60 degrés.

Chaque nervure 26 est droite et orientée longitudinalement comme l'orientation axiale de la paroi latérale 16, c'est-à-dire ici sensiblement verticalement.

Chaque nervure 26 prend racine dans la portion intermédiaire 23 formant épaulement et s'étend en direction de la paroi de fond 15 jusqu'à une extrémité distale 27 située à proximité de la paroi de fond 15.

Au niveau de la portion intermédiaire 23, chaque nervure 26 a une hauteur sensiblement égale à la largeur de l'épaulement, puis conserve une hauteur constante depuis la portion intermédiaire 23 jusqu'à mi-longueur environ, puis diminue de hauteur jusqu'à son extrémité distale 27 où la nervure 26 est alors à fleur de la face externe 20.

On notera ici que grâce à la nature rigide de la matière formant le godet 5, il est possible de ménager les nervures 26 sur la paroi ajourée 30 elle-même, alors que ceci n'aurait pas été possible avec un filtre en textile non-tissé.

Le godet 5 présente en outre deux doigts de verrouillage 25, saillant de part et d'autre de la portion supérieure 21 et ici situés à proximité de l'ouverture 14.

Ces doigts de verrouillage 25 forment avec la partie de la portion supérieure 21 qui les porte une interface mécanique de connexion 40, pour connecter le dispositif de collecte 1 au vagin artificiel.

Plus généralement, la portion supérieure 21 et les doigts de verrouillage 25 forment un membre d'entrée 52 du dispositif de collecte 1, comportant l'interface mécanique de connexion 40 et délimitant l'ouverture 14.

On notera que la paroi ajourée 30 forme un filtre associé à ce membre d'entrée 52 et délimitant avec le membre d'entrée 52 une chambre d'entrée 41 (figure 4) pour la semence dans le dispositif de collecte 1 ; laquelle chambre d'entrée 41 s'étend du côté d'une première face du filtre tournée vers l'ouverture 14.

La chambre d'entrée 41 correspond ici à l'espace interne du godet 5, tandis que la première face du filtre correspond ici à la face interne de la paroi ajourée 30.

Comme montré sur les figures 1 et 2, le godet 5 est partiellement reçu dans le sachet 4, au travers de son ouverture 6, sur une profondeur telle que la portion inférieure 22 est entièrement reçue dans le sachet 4.

En particulier, la paroi ajourée 30 est entièrement reçue dans le sachet 4.

On notera que la paroi ajourée 30 délimite avec le sachet 4 une chambre de recueil 42 pour la semence filtrée, laquelle chambre de recueil 42 s'étend du côté d'une seconde face du filtre (que forme la paroi ajourée 30) tournée vers la zone d'extrémité distale 8 du sachet 4.

La seconde face du filtre correspond ici à la face externe de la paroi ajourée 30, c'est à dire la face ici formée par la face externe 18 de la paroi de fond 15 et par la partie de la face externe 20 de la paroi latérale 16 située sur sa portion inférieure 22.

La portion supérieure 21 est quant à elle engagée dans la zone d'extrémité proximale 7 du sachet 4, en particulier dans l'ouverture 6, de sorte à maintenir le sachet 4 ouvert.

On notera que le diamètre externe moyen de la portion supérieure 21 correspond sensiblement au diamètre interne du sachet 4.

On notera encore que puisque la partie de la face externe 20 située dans la portion inférieure 22 est globalement en retrait (vers l'intérieur du godet 5) par rapport à la partie de la face externe 20 située dans la portion supérieure 21, la chambre de recueil 42 présente un espace 33 s'étendant entre la partie de la face externe 20 située dans la portion inférieure 22 et la face interne 11 du sachet 4.

La portion supérieure 21 présente ici une partie externe 31 qui saille extérieurement au-delà de l'extrémité 7 du sachet 4 et une partie interne 32 reçue dans le sachet 4.

Les doigts de verrouillage 25 sont portés par la partie externe 31.

Au niveau de la partie interne 32, la face interne 11 du sachet 4 est fixée à la face externe 20 de la paroi latérale 16, c'est-à-dire au pourtour externe du membre d'entrée 52, ici par soudure thermique.

On notera que la matière thermoplastique des films formant le sachet 4 comporte ici un polymère de type polyoléfine, qui a été sélectionné afin d'être miscible chimiquement avec le polypropylène formant le godet 5.

Le dispositif de collecte 1 comporte ici en outre un sachet de protection 36, similaire au sachet de collecte 4 et recouvrant ce dernier pour le protéger des éventuelles souillures, en particulier des contaminations bactériennes.

Le sachet de collecte 4 est ici entièrement reçu dans le sachet de protection 36.

Le sachet 36 est fixée à la portion supérieure 21 du godet 5 de la même manière que le sachet de collecte 4, la soudure entre le sachet 36 et la portion supérieure 21 étant réalisée entre l'extrémité 7 du sachet de collecte 4 et l'extrémité 12 du godet 5.

On notera que la matière formant les sachets 4 et 36 est choisie de sorte à être suffisamment translucide ou transparente afin de pouvoir juger de la qualité de la semence (visualiser d'éventuelles impuretés ou traces de sang) et/ou visualiser le niveau de remplissage du godet 5 ou du sachet 4 avec la semence.

Pour collecter de la semence, le dispositif de collecte 1 est raccordé à la sortie du vagin artificiel grâce à l'interface de connexion 40 du godet 5.

Le vagin artificiel est monté sur la verge d'un animal et actionné pour le stimuler.

La semence quittant le vagin artificiel entre par l'ouverture 14 et s'écoule dans la chambre d'entrée 41, qu'elle remplit partiellement, puis traverse la paroi ajourée 30 et est recueillie dans la chambre de recueil 42.

Le volume de semence produit par l'animal (l'éjaculat) est ainsi progressivement filtré par la paroi ajourée 30.

Au cours de la filtration, la substance « tapioca » a tendance à s'accumuler au fond du godet 5 et à boucher les orifices 24 de la paroi de fond 15, de sorte qu'à partir d'un certain moment la semence, et plus précisément sa fraction liquide, peine à s'écouler au travers des orifices 24 de la paroi de fond 15. Les orifices 24 de la paroi latérale 16 permettent à la fraction liquide de la semence de continuer à s'écouler au travers de la paroi ajourée 30 dans les meilleures conditions.

La semence filtrée par la paroi latérale 16 s'écoule ainsi dans l'espace 33 avant de tomber au fond du sachet 4.

On notera que grâce à l'espace 33, on diminue le risque que les films du sachet 4 viennent se plaquer contre la paroi ajourée 30 et colmater les orifices 24.

Les nervures 26, qui saillent dans l'espace 33, permettent de diminuer encore plus ce risque en maintenant les films du sachet 4 à l'écart de la face externe 20 même lorsque ces films ont tendance à s'en rapprocher, par exemple si le godet 5 est incliné.

Grâce à l'espace 33 et aux nervures 26, il est ainsi possible de conférer à la paroi ajourée 30 une forme quasiment cylindrique sans risquer de colmater les orifices 24 de la paroi ajourée 30.

Comparée à une forme conique, cette forme cylindrique offre une plus grande surface pour ménager les orifices 24 dans la paroi latérale 16 et dans la paroi de fond 15 du godet 5, les orifices 24 pouvant être ainsi plus nombreux que sur une paroi similaire mais conique.

La forme cylindrique permet en outre que le volume délimité par la paroi ajourée 30 soit plus grand que si cette dernière était conique. De cette manière, une plus grande quantité de « tapioca » peut être reçue dans le godet 5 tout en conservant suffisamment d'orifices 24 non bouchés sur la paroi latérale 16 pour permettre à la fraction liquide de s'écouler.

Par ailleurs, la demanderesse a constaté que, dans certaines conditions, une telle paroi ajourée 30 permettait d'obtenir des performances exceptionnelles quant à la vitesse de filtration de la semence, en comparaison avec la poche de filtration en textile non-tissé du dispositif de collecte décrit dans la demande internationale WO 2006/131781.

Par exemple, lors de tests effectués avec de la semence de verrats de la race des piétrains, la demanderesse a constaté que pour un volume donné de semence à filtrer aboutissant à un volume de semence filtrée récolté compris entre 300 et 400 ml, lorsque le temps de filtration est compris entre 2 et 4 minutes lorsqu'on utilise un dispositif de collecte ayant une poche de filtration en textile non-tissé, ce temps de filtration est compris entre 15 et 60 secondes lorsqu'on utilise le dispositif de collecte 1 pourvu de la paroi ajourée 30.

En outre, le volume de la fraction liquide de semence retenue par mouillage (et donc non recueillie dans le sachet 4) est bien moindre lorsqu'on filtre cette semence avec la paroi ajourée 30 que lorsqu'on la filtre avec la poche en textile non-tissé.

Les tests menés par la demanderesse ont en effet montré que, pour un volume donné de semence à filtrer, lorsqu'entre 7 % et 8 % de la fraction liquide de ce volume (c'est-à-dire la partie du volume de semence sans la substance « tapioca ») sont retenus par mouillage sur la poche en textile non-tissé, alors moins d'1 % de la fraction liquide de ce volume est retenu par la paroi ajourée 30.

En outre, la concentration et la motilité des spermatozoïdes ne sont pas altérées par la paroi ajourée 30 et sont au contraire similaires à celles qu'on observe lorsque la semence est filtrée par la poche de filtration en textile non-tissé.

On notera encore que le polypropylène formant le godet 5 présente une tension de surface relativement faible, ici d'environ 29 dynes, ce qui confère des propriétés hydrophobes à la surface de la paroi ajourée 30 et favorise l'écoulement de la semence au travers des orifices 24.

En pratique, la matière dont est fait le godet 5 peut être choisie de sorte à présenter une tension de surface inférieure à 30 dynes.

Une fois que l'opération de collecte est terminée, le sachet 36 est retiré et la portion du sachet 4 dans laquelle est reçue la semence filtrée peut alors être séparée du reste du dispositif 1, par exemple en déchirant les films, et emportée dans un laboratoire d'analyses et de conditionnement, où la semence est analysée, diluée, avant d'être conservée dans des sachets de conditionnement unitaires.

Afin de faciliter le déchirement des films, le sachet de collecte 4 et/ou de protection 36 peuvent être chacun pourvu d'une encoche (non illustrée), ménagée dans les portions soudées des films de sorte à y former une prédécoupe.

En variante, les sachets 4 et/ou 36 sont opaques à la lumière, ou à certaines longueurs d'ondes du rayonnement électromagnétique spécifiques néfastes à la conservation de la semence, par exemple les rayons UV et/ou IR.

A cet effet, des pigments de protection contre de tels rayons peuvent être introduits dans la matière dont sont faits les sachets 4 et 36 et/ou peuvent être imprimés à la surface de cette matière.

Dans d'autres variantes non illustrées :
- la matière thermoplastique dont est fait le godet est dépourvue de polypropylène et comporte une autre matière, par exemple du polyéthylène ;
- la matière thermoplastique dont est fait le godet n'est pas un polymère de type polyoléfine mais d'un autre type, par exemple de type polyacétal ;
- la matière dont est fait le godet n'est pas thermoplastique mais d'une autre nature, par exemple métallique, tel qu'en acier inox, le godet étant alors réalisée par emboutissage et perçage ;
- la soudure entre le sachet et le godet n'est pas thermique mais est faite différemment, par exemple par ultrasons, ou par laser avec l'une des matières du godet et du sachet qui est transparente à la longueur d'onde du laser tandis que l'autre des matières est rendue opaque à cette longueur d'onde, par exemple à l'aide de pigments absorbant cette longueur d'onde incorporés dans cette autre matière ;
- le sachet et le godet ne sont pas soudés mais plutôt fixés par collage, ou à l'aide d'un élastique annulaire serrant le sachet sur le godet ;
- la paroi latérale du godet présente une section elliptique, rectangulaire, ou carrée, plutôt que circulaire ;
- la paroi de fond du godet n'est pas plate mais par exemple incurvée, ou conique ;
- l'une de la paroi latérale et de la paroi de fond du godet est dépourvue d'orifices ;
- le premier motif prédéterminé comporte plus ou moins de vingt-quatre colonnes d'orifices, ces orifices sont agencés autrement que selon un quadrillage de rectangles, par exemple selon un quadrillage de carrés, ou sont agencés en quinconce ;
- selon le deuxième motif prédéterminé, les orifices sont agencés autrement qu'en quinconce, par exemple en un quadrillage de rectangles, ou de carrés ;
- la portion cylindrique des orifices est tournée vers l'extérieur du godet, tandis que la portion en entonnoir est tournée vers l'intérieur du godet et s'évase vers l'intérieur du godet ;
- la surface délimitant les orifices est dépourvue d'une portion en entonnoir et est uniquement cylindrique, ou au contraire est dépourvue d'une portion cylindrique et est uniquement en entonnoir ;
- la surface délimitant les orifices a en section une forme autre que circulaire, par exemple ovale, rectangulaire ou triangulaire, pour une section de passage comparable ;
- le godet est dépourvu d'une portion intermédiaire formant épaulement ;
- le godet présente plus, ou moins, de six nervures sur sa portion inférieure, le pas angulaire prédéterminé selon lequel les nervures sont réparties étant adapté en conséquence ;
- les nervures sont configurées différemment, et sont par exemple annulaires et orientées selon un plan perpendiculaire à la direction d'extension longitudinale de la paroi latérale tubulaire ;
- les nervures ne sont pas continues mais interrompues et/ou sont de hauteur constante sur toute leur longueur ;
- l'interface de connexion est dépourvue de doigts de verrouillage et comporte par exemple à la place un filetage pour visser le godet dans le col de sortie du vagin artificiel ; et/ou
- le dispositif de collecte est dépourvu d'un sachet de protection.

De nombreuses autres variantes sont possibles en fonction des circonstances, et l'on rappelle à cet égard que l'invention ne se limite pas aux exemples décrits et représentés.

## Revendications

1. Dispositif pour la collecte de semence animale, comportant :
- un membre d'entrée (28) comportant une interface mécanique de connexion (40) à un vagin artificiel et délimitant une ouverture d'entrée (14) dans le dispositif de collecte (1) ;
- un filtre associé au membre d'entrée (28) et délimitant avec le membre d'entrée (28) une chambre d'entrée (41) s'étendant du côté d'une première face (17, 19) du filtre tournée vers l'ouverture d'entrée (14) ; et
- un sachet de collecte (4) s'étendant entre une zone d'extrémité distale (8) fermée et une zone d'extrémité proximale (7) présentant une ouverture (6) dans laquelle est engagé ledit membre d'entrée (28) ; avec ledit sachet (4) qui est fixé audit membre d'entrée (28) ; et avec ledit sachet (4) et ledit filtre qui délimitent une chambre de recueil (42) s'étendant du côté de la seconde face (18) du filtre tournée vers la zone d'extrémité distale (8) fermée du sachet (4) ;
grâce à quoi de la semence entrant dans le dispositif (1) par l'ouverture d'entrée (14) traverse le filtre et est recueillie dans la chambre de recueil (42) ;
**caractérisé en ce que** le filtre est formé par une paroi ajourée (30) venue de matière avec le membre d'entrée (28).

2. Dispositif selon la revendication 1, **caractérisé en ce que** ladite paroi ajourée (30) présente une portion tubulaire (22) s'étendant axialement entre une première extrémité (49) tournée vers ladite zone d'extrémité proximale (7) dudit sachet (4) et une deuxième extrémité (50) tournée vers ladite zone d'extrémité distale (8) fermée dudit sachet (4), et une portion de fond (15) s'étendant transversalement à ladite portion tubulaire (22) et fermant cette dernière à sa deuxième extrémité (50).

3. Dispositif selon la revendication 2, **caractérisé en ce que** ladite portion tubulaire (22) a en section une forme circulaire.

4. Dispositif selon l'une quelconque des revendications 2 ou 3, **caractérisé en ce que** ladite portion tubulaire (22) présente une face externe (20) nervurée.

5. Dispositif selon la revendication 4, **caractérisé en ce que** ladite face externe (20) nervurée présente des nervures (26) droites orientées longitudinalement comme l'orientation axiale de la portion tubulaire (22).

6. Dispositif selon la revendication 5, **caractérisé en ce que** lesdites nervures (26) sont réparties autour de la portion tubulaire (22) selon un pas angulaire prédéterminé.

7. Dispositif selon l'une quelconque des revendications 1 à 6, **caractérisé en ce que** ladite paroi ajourée (30) est ajourée par une pluralité d'orifices (24) ayant un diamètre minimal prédéterminé compris entre 0,5 mm et 1,5 mm, lesdits orifices (24) étant répartis sur ladite paroi ajourée (30) en étant agencés selon un motif prédéterminé procurant une densité surfacique d'orifices (24) comprise entre 3 et 16 orifices par cm².

8. Dispositif selon l'une quelconque des revendications 1 à 7, **caractérisé en ce que** ladite paroi ajourée (30) est ajourée par une pluralité d'orifices (24) et présente une portion tubulaire (22) s'étendant axialement entre une première extrémité (49) tournée vers ladite zone d'extrémité proximale (7) dudit sachet (4) et une deuxième extrémité (50) tournée vers ladite zone d'extrémité distale (8) fermée dudit sachet (4), et une portion de fond (15) s'étendant transversalement à ladite portion tubulaire (22) et fermant cette dernière à sa deuxième extrémité (50), lesdits orifices (24) étant répartis sur ladite paroi ajourée (30) en étant agencés sur ladite portion tubulaire (22) selon un premier motif prédéterminé et sur ladite portion de fond (15) selon un deuxième motif prédéterminé, lesdits premier motif prédéterminé et deuxième motif prédéterminé procurant une densité surfacique d'orifices (24) plus importante sur ladite portion de fond (15) que sur ladite portion tubulaire (22).

9. Dispositif selon la revendication 8, **caractérisé en ce que** ledit premier motif prédéterminé procure une densité surfacique d'orifices (24) sur la portion tubulaire (22) comprise entre 3 et 7 orifices par cm².

10. Dispositif selon l'une quelconque des revendications 8 ou 9, **caractérisé en ce que** ledit deuxième motif prédéterminé procure une densité surfacique d'orifices (24) sur la portion de fond (15) comprise entre 10 et 16 orifices par cm².

11. Dispositif selon l'une quelconque des revendications 8 à 10, **caractérisé en ce que**, selon au moins l'un desdits premier motif prédéterminé et deuxième motif prédéterminé, lesdits orifices (24) sont agencés en un quadrillage de rectangles.

12. Dispositif selon l'une quelconque des revendications 8 à 11, **caractérisé en ce que**, selon au moins l'un desdits premier motif prédéterminé et deuxième motif prédéterminé, lesdits orifices (24) sont agencés en quinconce.

13. Dispositif selon l'une quelconque des revendications 8 à 12, **caractérisé en ce que** chaque dit orifice (24) est délimité par une surface en entonnoir (35) augmentant de diamètre vers la seconde face (18) du filtre.

14. Dispositif selon l'une quelconque des revendications 1 à 13, **caractérisé en ce que** ledit membre d'entrée (28) et ladite paroi ajourée (30) font partie d'une pièce en matière thermoplastique moulée par injection.

15. Dispositif selon l'un quelconque des revendications 1 à 14, **caractérisé en ce qu'**il comporte un sachet de protection (36) qui recouvre ledit sachet de collecte (4) et qui est fixé audit membre d'entrée (28).
